**Europäisches Patentamt**

**European Patent Office**

**Office européen des brevets**

(11) Veröffentlichungsnummer : **0 355 480 B1**

(12) # EUROPÄISCHE PATENTSCHRIFT

(45) Veröffentlichungstag der Patentschrift : **10.06.92 Patentblatt 92/24**

(51) Int. Cl.$^5$ : **C07C 231/02, C07C 233/65**

(21) Anmeldenummer : **89114066.7**

(22) Anmeldetag : **29.07.89**

(54) **Verfahren zur Herstellung von N-Methylbenzamid.**

(30) Priorität : **13.08.88 DE 3827531**

(43) Veröffentlichungstag der Anmeldung : **28.02.90 Patentblatt 90/09**

(45) Bekanntmachung des Hinweises auf die Patenterteilung : **10.06.92 Patentblatt 92/24**

(84) Benannte Vertragsstaaten : **DE ES FR GB**

(56) Entgegenhaltungen : **US-A- 3 763 234**

(56) Entgegenhaltungen :
**ANGEWANDTE CHEMIE, 98. Jahrgang, Heft Nr. 6, 1986, Seiten 569-570ff, VCH Verlagsgesellschaft mbH, Weinheim, D; K. MATSUMOTOet al: "Direkte Aminolyse von nicht aktivierten Estern bei hohem Druck"**
**PATENT ABSTRACTS OF JAPAN, Band 3, Nr. 100 (C-56), 24. August 1979, 114 C 56; & JP-A-54 79 241**
**BEILSTEINS HANDBUCH DER ORGANISCHEN CHEMIE, 4. Auflage, 9. Band, 1926, Seite 201, Verlag Julius Springer**
**BEILSTEINS HANDBUCH DER ORGANISCHEN CHEMIE, 4. Auflage, 2. Ergänzungswerk, 9. Band, 1949, Seite 165, Springer Verlag**

(73) Patentinhaber : **BAYER AG**
**W-5090 Leverkusen 1 Bayerwerk (DE)**

(72) Erfinder : **Endres, Robert, Dr.**
**Kaltmüntener Strasse 8**
**W-5060 Bergisch-Gladbach 2 (DE)**

## Beschreibung

Die vorliegende Erfindung betrifft ein verbessertes Verfahren zur Herstellung von N-Methylbenzamid aus Benzoesäuremethylester und Methylamin.

Amide von Arylcarbonsäuren erhält man, indem man auf die entsprechenden Ester, Säurenchloride oder Anhydride Ammoniak bzw. Amine einwirken läßt. Die Synthese über die Ester hat im Vergleich zu der über die Säurechloride den Vorteil, daß keine freiwerdende Säure mit einer Teilmenge Ammoniak bzw. Amin neutralisiert werden muß. Allerdings ist die Umsetzung von nicht aktivierten Estern mit Ammoniak bzw. primären Aminen erschwert und erfordert Temperaturen von über 200°C. Dieses Problem kann man entweder dadurch beheben, daß man bei hohem Druck (einige Kbar) arbeitet, wie z.B. nach K. Matsumoto, S. Hashimoto und S. Otani, Angew. Chem. 98 (1986), Nr. 6, S. 569 f. Oder man verwendet saure Katalysatoren wie z.B. in der japanischen Offenlegungsschrift Nr. 54-79241 vom 25.06.1979 beschrieben, nach der Ammoniumchlorid die Aminolyse von p-Methylbenzoesäure im Temperaturbereich von 50 bis 160°C ermöglicht. Dieses Verfahren hat jedoch den Nachteil, daß Ammoniumchlorid korrosiv wirkt und bei Chromnickelstählen, die normalerweise für Aminolysen eingesetzt werden, Lochfraß verursacht. Darüberhinaus wird nach der säurekatalysierten Methode z.B. Benzoesäuremethylester nicht vollständig mit Methylamin umgesetzt, wie die Vergleichsbeispiele 1 und 2 dieser Schrift (ohne und mit Toluol als Reaktionsmedium) zeigen. Man findet im Endprodukt als Ausgangsrohstoff noch so viel Benzoesäuremethylester, daß für viele Einsatzzwecke ein zusätzliches Reinigungsverfahren notwendig wäre.

Beim erfindungsgemäßen Verfahren zur Herstellung von N-Methylbenzamid aus dem preisgünstigen Rohstoff Benzoesäuremethylester treten diese Nachteile nicht auf. Es ist dadurch gekennzeichnet, daß die Reaktion in Methanol mit überstöchiometrischer Methylaminmenge im Temperaturbereich von 60 bis 150°C bei 2 bis 12 bar, vorzugsweise 4-8 bar, durchgeführt wird.

Als Medium für diese Reaktion hat sich Methanol als vorteilhaft erwiesen, da es auch bei der Reaktion von Methylamin mit Benzoesäuremethylester entsteht, so daß das Reaktionsmedium einheitlich bleibt. Andere Lösungsmittel wie z.B. Toluol verhalten sich dagegen nachteilig, wie das Vergleichsbeispiel 2 zeigt. Für das beanspruchte Verfahren wird ein Methanolzusatz zwischen 1 und 2 Mol, bezogen auf 1 Mol eingesetzten Benzoesäuremethylester, bevorzugt. Größere Mengen sind nicht nötig, da sie die Produktaufarbeitung und das spezifische Reaktionsvolumen belasten würden.

Der Überschuß von Methylamin, bezogen auf 1 Mol Benzoesäuremethylester, sollte bevorzugt 0,5 bis 2 Mol betragen, damit die Bildung von N-Methylbenzamid vollständig wird. Größere Aminüberschüsse sind zwar nicht für die Umsetzung, jedoch für die Produktaufarbeitung nachteilig. Die bevorzugte Reaktionstemperatur liegt zwischen 60 und 150°C, wobei die tieferen Temperaturen längere Reaktionszeiten erfordern. Andererseits ist der Eigendruck insbesondere zu Beginn der Reaktion bei höheren Temperaturen größer. Besonders bevorzugt für die erfindungsgemäße Synthese arbeitet man zwischen 80 und 100°C, wobei der Eigendruck zu Synthesebeginn zwischen 4 und 8 bar liegt und sich nach 5 bis 12 Stunden konstant auf ein niedrigeres Niveau entsprechend dem Verbrauch an Benzoesäuremethylester und Methylamin einstellt, um unumgesetzten Benzoesäuremethylester in den Spurenbereich zu drücken. N-Methylbenzamid fällt dann so rein an, daß praktisch kein Ester mehr nachgewiesen werden kann.

Entfernt man destillativ überschüssiges Methylamin und teilweise Methanol, so fällt N-Methylbenzamid so rein an, daß es in vielen Fällen in einer Eintopfreaktion direkt weiterverwendet werden kann, wie z.B. für die Umsetzung mit Chlorsilanen zu Amidosilanen nach DE-AS 3 504 644. Auch eine direkte Weiterreaktion zu Farbstoffen und Wirkstoffen ist möglich. Isoliert man das so hergestellte N-Methylbenzamid, so fallen im Gegensatz zum Verfahren nach den Vergleichsbeispielen völlig farblose und sehr reine Kristalle an.

Das abdestillierte Gemisch von Methylamin und Methanol kann direkt in die nächste Synthese rückgeführt werden, so daß nur verbrauchtes Methylamin aufgestockt werden muß. Da sich Methylamin bei R.T. zu 36,4 Gew.-% in Methanol löst, kann Methanol als Waschflüssigkeit in einem der Syntheseapparatur nachgeschalteten Gaswäscher verwendet werden. Der Inhalt des Wäschers kann vorteilhaft periodisch in die Synthese rückgeführt werden.

Die folgenden Beispiele sollen die vorliegende Erfindung veranschaulichen.

Beispiel 1 (Vergleich)

In einem 0,8 l Stahlautoklaven (Werkstoffnr. 1.4571) wurden bei R.T. 1 Mol Benzoesäuremethylester und 0.06 Mol Ammoniumchlorid vorgelegt. Der Autoklav wurde evakuiert und anschließend wurden 2,5 Mol Methylamin eingesaugt. Die Mischung wurde unter Rühren auf 100°C erhitzt, wobei sich ein Überdruck von 7 bar einstellte. Die Reaktion konnte über die Druckabnahme verfolgt werden und war nach 5 h beendet. Nach Abkühlung und Druckausgleich wurde das Produkt im Vakuum vom entstandenen Methanol und überschüssigen Methylamin befreit. Man erhielt gelbliche Kristalle vom Schmelzpunkt 78-79°C. Ausbeute: 91 %. GC-Analyse der Nebenbestandteile: 2,5 % Benzoesäuremethylester und 0,8 % Benzoesäure.

Nach 5 Reaktionsansätzen konnte an der Werkstoffoberfläche Lochfraß beobachtet werden.

## Beispiel 2 (Vergleich)

In einem 1 l Stahlautoklaven (Werkstoffnr. 1.4571) wurden bei R.T. 1 Mol Benzoesäuremethylester, 0,06 Mol Ammoniumchlorid und 400 g Toluol vorgelegt. Die Mischung wurde wie im Vergleichsbeispiel 1 mit 2,5 Mol Methylamin 5 h bei 100°C zur Reaktion gebracht und analog aufgearbeitet. Es wurden gelbliche Kristalle vom Schmelzpunkt 77-78°C erhalten. Ausbeute: 89 %. Gc-Analyse der Nebenbestandteile: 6,9 % Benzoesäuremethylester und 0,5 % Benzoesäure

Ebenso wie beim Vergleichsbeispiel 1 stellte sich nach mehreren Versuchen an der Werkstoffoberfläche Lochfraß ein.

## Beispiel 3 (erfindungsgemäß)

In einem 6 l Stahlautoklaven (Werkstoffnr. 1.4571) wurden bei R.T. 15,64 Mol Benzoesäuremethylester vorgelegt, anschließend wurde der Autoklav evakuiert und 24 Mol gasförmiges Methylamin eingeleitet, wobei der Autoklavendruck auf 5 bar anstieg. Die Umsetzung wurde bei 90° C durchgeführt, wobei der Überdruck auf 6 bar anstieg und während der Reaktion auf 4 bar abnahm. Nach 6 h war die Reaktion beendet (Druckkonstanz); das Produktgemisch wurde noch 2 h nachgerührt, auf R.T. abgekühlt und nach dem Druckausgleich eine Probe zur analytischen Charakterisierung entnommen. Man erhielt farblose Kristalle vom Schmelzpunkt 80-81°C. GC-Analyse der Syntheselösung:

| | |
|---|---|
| Methylamin | 23,6 % |
| Methanol | 38,0 % |
| Benzoesäure | n.n. % |
| N-Methylbenzamid | 38,2 % |
| Benzoesäuremethylester | n.n % |

n.n.= nicht nachweisbar

Die GC-Analyse im Spurenbereich ergab 7 ppm Benzoesäuremethylester.

Die Produktlösung wurde in ein 6 l-Rührgefäß mit aufgesetztem fallenden Kühler und Wechselvorlage unter $N_2$-Überlagerung überführt und bei Normaldruck bis 80°C Sumpftemperatur Methylamin und Methanol abgezogen. Die so erhaltene methanolische Methylaminlösung (ca. 1,5 l) konnte in die nächsten Syntheseansätze rückgeführt werden.

Nach mehr als 20 Synthesen wurde auf der Werkstoffoberfläche kein Lochfraß beobachtet.

## Patentansprüche

1. Verfahren zur Herstellung von N-Methylbenzamid aus Benzoesäuremethylester, dadurch gekennzeichnet, daß die Reaktion in Methanol mit Überstöchiometrischer Methylaminmenge im Temperaturbereich von 60 bis 150°C bei 2 bis 12 bar durchgeführt wird.

2. Verfahren nach Anspruch 1, dadurch gekennzeichnet, daß die Methanolmenge 1 bis 2 Mol (bezogen auf 1 Mol Ester) beträgt.

3. Verfahren nach den Ansprüchen 1 oder 2, dadurch gekennzeichnet, daß Methylamin in einer überstöchiometrischen Menge zwischen 50 und 200 Mol.-% (bezogen auf 1 Mol Ester) eingesetzt wird.

## Claims

1. A process for the production of N-methyl benzamide from benzoic acid methyl ester, characterized in that the reaction with methanol is carried out with an overstoichiometric quantity of methyl amine at a temperature of 60 to 150°C under a pressure of 2 to 12 bar.

2. A process as claimed in claim 1, characterized in that the methanol is used in a quantity of 1 to 2 mol (based on 1 mol ester).

3. A process as claimed in claim 1 or 2, characterized in that the methyl amine is used in an overstoichiometric quantity of 50 to 200 mol-% (based on 1 mol ester).

**Revendications**

1. Procédé pour la fabrication du N-méthylbenzamide à partir du benzoate de méthyle, caractérisé en ce que la réaction avec un excès de méthylamine sur la quantité stoechiométrique est mise en oeuvre dans le méthanol dans l'intervalle de températures de 60 à 150°C sous 2 à 12 bar.

2. Procédé selon la revendication 1, caractérisé en ce que la quantité de méthanol est de 1 à 2 mol (rapportée à 1 mol d'ester).

3. Procédé selon la revendication 1 ou 2, caractérisé en ce que la méthylamine est utilisée en excès entre 50 et 200 mol% de la quantité stoechiométrique (rapportée à 1 mol d'ester).